# EUROPEAN PATENT SPECIFICATION

(11) **EP 0 721 782 B1**
(45) Date of publication and mention of the grant of the patent: **16.08.2000**
(21) Application number: 96300197.9
(22) Date of filing: 09.01.1996
(51) Int. Cl.: A61K 39/39, A61K 39/145, A61K 39/10, C07K 14/235, C07K 14/11

(54) **Influenza vaccine containing pertussis toxin**
Pertussistoxin enthaltender Influenzaimpfstoff
Vaccin contre la grippe contenant de la toxine de pertussis

(30) Priority: 16.01.1995 GB 9500788
(43) Date of publication of application: 17.07.1996
(73) Proprietor: Medeva Europe Limited, Leatherhead, Surrey KT22 7PQ (GB)
(72) Inventor: Roberts, Mark, Department of Veterinary Pathology, Glasgow G61 1QH (GB)
(74) Representative: Hutchins, Michael Richard

(56) References cited:
- EP-A- 0 462 534
- WO-A-95/34323
- VACCINE, vol. 12, no. 14, 1994, pages 1255-1258, XP000612060 T. OKA ET AL: "Enhancing effects of pertussis toxin B oligomer on the immunogenicity of influenza vaccine administered intranasally"
- INFECTION AND IMMUNITY , vol. 63, no. 6, June 1995, USA, pages 2100-2108, XP000612059 M. ROBERTS ET AL: "A mutant Pertussis Toxin Molecule that lacks ADP-Ribosyltransferase Activity, PT-9K/129G, is an Effective Mucosal Adjuvant for Intranasally Delivered Proteins "
- INFECTION AND IMMUNITY, vol. 61, no. 7, July 1993, USA, pages 2834-2840, XP000612058 HONG-HUA MU ET AL: "Enhancement of Interleukin-4 Production by Pertussis Toxin"
- PATENT ABSTRACTS OF JAPAN vol. 15, no. 345 (C-0864), 3 September 1991 & JP 03 135923 A (CHEMO SERO THERAPEUT RES INST), 10 June 1991,
- W. J. BLACK ET AL: "ADP-ribosyltransferase Acxtivity of Pertussis Toxin and Immunomodulation by Bordetella pertussis", SCIENCE, , , vol. 240, no. , pages 656 to 658
- N. LYCKE TE AL: "The adjuvant effect of Vibrio cholerae and Escherichia coli heat-labile enterotoxins is linked to their ADP-ribosyltransferase activity", EUR. J. IMMUNOL., , , vol. 22, no. , pages 2277 to 2281

## Description

The invention relates to a vaccine composition comprising influenza virus antigens and an adjuvant.

Current influenza vaccines consist of either inactivated whole virus, disrupted virus (split vaccines) or purified preparations of the membrane glycoproteins haemagglutinin (HA) and neuraminidase (NA) sub-unit vaccines.

Haemagglutinin and neuraminidase are the antigens to which protective antibody responses are directed, haemagglutinin being the major protective antigen. Estimates of the efficacy of these parenterally administered vaccines vary greatly. Such vaccines are believed to act primarily by eliciting circulating anti-haemagglutinin IgG antibodies that transudate into the lower respiratory tract.

M. L. Clements *et al*, J. Clinical Microbiology 24, 157-160, 1986, have previously reported that both secretory IgA and serum IgG participate in immunity to influenza virus. Moreover, in mice, a number of published studies have demonstrated the importance of locally synthesised IgA to protection against influenza infection. It has also been found that an advantage of stimulating a local IgA response to influenza is that it is often of a broader specificity than the serum response and thus can provide cross-protection against viruses possessing haemagglutinin molecules different from those present in the vaccine. Accordingly, influenza vaccines that elicit both local secretory and serum anti-haemagglutinin responses should provide superior immunity to current vaccines. However, parenteral vaccination (intramuscular, sub-cutaneous etc) is not effective at eliciting local antibody production, if there has been no previous mucosal exposure (e.g. infection). In order to achieve efficient stimulation of the mucosa-associated lymphoid tissue (MALT), the immunogen needs to be applied topically to the mucosal surface during the course of vaccination.

Mucosal administration of influenza vaccine would have a number of advantages over traditional parenteral immunisation regimes. Paramount amongst these are more effective stimulation of the local mucosal immune system of the respiratory tract and the likelihood that vaccine uptake rates would be increased because the fear and discomfort associated with injections would be avoided. Accordingly, a number of attempts have been made to develop mucosal influenza vaccines. A drawback however is that most non-replicating immunogens are poorly immunogenic when ingested or inhaled, and soluble proteins are particularly inefficient mucosal immunogens. This is undoubtedly because the non-specific defence mechanisms of the mucous membranes (e.g. resident macrophages, lysozyme, extremes of pH, bile acids, digestive enzymes, mucus, shedding of epithelial cells, flushing mechanisms such as peristalsis, ciliary beating, micturation, etc) will readily denature, degrade and eliminate most soluble proteins resulting in the MALT encountering minute quantities of such immunogens.

Administering large repeated doses of a particular protein may be expected to enhance the immune response. However, the result of such immunisation is often the induction of a state of immunological unresponsiveness known as oral tolerance where the individual responds poorly to subsequent parenteral immunisation with the same antigen (it would be more accurately labelled mucosal tolerance because inhalation of large amounts of soluble proteins also induces this state). The regulatory mechanisms involved in initiation of oral tolerance are poorly understood but are believed to have evolved to prevent animals developing inappropriate and possibly deleterious immune responses to environmental and dietary proteins. One of the major goals of modern vaccinology is to devise means of eliciting strong mucosal and systemic immune responses to soluble proteins whilst avoiding the induction of mucosal tolerance.

Although many soluble proteins are poor mucosal immunogens, some proteins, particularly microbial components that can recognise and bind to receptors on the surface of eucaryotic cells, can elicit local and systemic immune responses. Cholera toxin (CT), for example, is one of the most potent mucosal immunogens known.

Some microbial components have also been reported as possessing mucosal adjuvant activity. Thus, cholera toxin is known to be a mucosal adjuvant that enhances the responses to some co-administered antigens. In mice only minute quantities of CT are necessary for the adjuvant effect. Unfortunately, a dose of 2µg of CT fed to human volunteers is diarrheaogenic and a dose of 5µg induced purging indistinguishable from classical cholera. Active CT is therefore clearly unacceptable as a mucosal adjuvant in humans.

Pertussis toxin (PTX) has also been reported to have adjuvant properties, but a major drawback, in addition to its inherent toxic properties, is its property of stimulating and enhancing IgE production thereby leading to anaphylaxis to co-administered proteins - see for example Mu et al Infection and Immunity, pp. 2834-2840, July 1993.

Cholera toxin, pertussis toxin and the structurally related E. coli heat labile toxin (LT), are examples of toxins which have a bipartite AB₅ structure consisting of a protomer (the A sub-unit) and a B pentamer (the B sub-unit). The A sub-unit is generally regarded as being responsible for the toxicity of the toxin whereas the B sub-unit is generally thought to be involved in binding to the cell surface. For example, in cholera toxin, the A sub-unit (CTA) is the enzymatic moiety of CT responsible for the covalent modification of host G proteins and the consequent toxicity of CT, while the B sub-unit (CTB) mediates the binding of CT to its receptor (ganglioside GM₁) on the surface of eucaryotic cells. CTB is non toxic and is also a good mucosal immunogen.

There have been reports that the B sub-units of certain of the bipartite microbial toxins have mucosal adjuvant properties. For example, Japanese patent application number 3-135923 (Zaidan Hojin) published 10th June 1991 discloses that the B sub-unit of pertussis toxin is an adjuvant for Influenza A Yamagata strain antigens when administered intranasally. The B sub-unit preparation was prepared from the whole toxin by urea treatment followed by column chromatography using a sepharose column. A similar disclosure can be found in the article by Oka *et al,* Vaccine, Vol. 12(14) 1994, pp. 1255-1258.

The cholera toxin B sub-unit (CTB) has also been investigated as a mucosal adjuvant by many groups, with conflicting results. CTB obtained from commercial suppliers is prepared from whole toxin and often contains trace quantities of CT which could be responsible for the adjuvanticity of CTB reported by some authors.

Furthermore, it has been found that recombinant CTB and the highly related E. coli heat labile toxin B pentamer (LTB), although immunogenic themselves, are devoid of adjuvanticity. Thus, Holmgren et al (Vaccine, Vol II, pp 1179-1184, 1993) have reported that when highly purified or recombinant CTB was used, they were consistently unable to observe an adjuvant action of CTB for other antigens admixed therewith. Holmgren et al concluded that the whole CT molecule is required for the adjuvant action. They also tested a mutant form of E. coli heat labile toxin (LT) in which the B sub-unit was identical to that of the normal B sub-unit, and the A sub-unit was identical with the exception of a single amino acid substitution in position 112 (Glu→Lys). However, the mutant form, which did not possess ADP-ribosylating activity and did not cause fluid secretion in rabbit ligated loops, failed to give rise to any significant IgA response against itself and demonstrated no adjuvant properties.

Attempts have also been made to use the B sub-unit of cholera toxin (CTB) as an adjuvant for mucosal influenza vaccines. However, such attempts, in common with other approaches such as encapsulating the antigens in microspheres, or using live attenuated strains, have been largely unsuccessful.

It has now been found that a mutant form of pertussis toxin is an excellent adjuvant for influenza antigens, as well as displaying good immunogenic properties itself. WO-A-95/34323, an earlier Application published after the priority date of the present invention, discloses the adjuvant properties of mutant pertussis toxins, but does not disclose compositions containing both influenza A and influenza B antigens.

Accordingly, in a first aspect, the invention provides a vaccine composition comprising an effective immunogenic amount of an influenza antigen and an effective adjuvant amount of a non-toxic mutant form of pertussis toxin, characterised in that both influenza A and influenza B viral antigens are present in the composition.

In a particular aspect, the invention provides a vaccine composition as hereinbefore defined, the vaccine composition being adapted for intranasal administration.

The influenza antigens preferably contain haemagglutinin and neuraminidase influenza virus antigens. In particular, the influenza antigens may comprise purified haemagglutinin and neuraminidase influenza virus antigens.

It is preferred that the purified haemagglutinin and neuraminidase antigens are present in the form of rosettes. The rosettes preferably are particles with a radius in the range of 10 to 25 nanometres.

It is preferred that the rosettes are substantially free of lipid and, moreover, it is preferred that the purified haemagglutinin and neuraminidase antigen preparation as a whole is substantially free of lipids.

An example of a haemagglutinin/neuraminidase preparation suitable for use in the compositions of the present invention is the "Fluvirin" product manufactured and sold by Evans Medical Limited of Speke, Merseyside, United Kingdom, and see also S. Renfrey and A. Watts, Vaccine, 1994, Volume 12, Number 8, pp 747-752.

The non-toxic mutant form of pertussis toxin preferably is one in which the Glu 129 amino acid in the S₁ sub-unit has been substituted by another amino acid (eg glycine) and/or the arginine 9 amino acid residue in the S₁ sub-unit has been substituted (eg by lysine). The mutant toxin preferably is a double mutant form; for example, a double mutant form in which both the Glu 129 amino acid and the arginine 9 amino acid residue in the S₁ sub unit have been substituted.

Particular examples of non-toxic double mutant pertussis toxins for use in the present invention are those disclosed in European Patent Application EP-A-0462534 (Sclavo SpA). A preferred non-toxic double mutant toxin is the mutant described in Example 1 of EP-A-0462534, in which the arginine 9 residue has been substituted by lysine, and the glutamic acid 129 residue has been substituted by glycine. This mutant is referred to hereinafter as PT 9K/129G.

The invention also provides the use of a non-toxic mutant form of pertussis toxin as hereinbefore defined for the manufacture of an adjuvant composition for stimulating or enhancing the protective immune response of an influenza antigen co-administered therewith.

The influenza antigen and the adjuvant pertussis mutant toxin, are preferably administered mucosally. Thus they may be administered orally, intranasally, intrabronchially or by any other mucosal route, but it is preferred that the influenza antigen and the mutant toxin be given by intranasal administration.

The influenza antigen and the adjuvant mutant toxin may be administered separately, for example, within a short period of one another, or they may be administered together simultaneously. When administered together, they may be formulated as a mixture of discrete entities. Alternatively, they may be chemically linked together, or may form part of a fusion protein produced by recombinant DNA methods.

The vaccine compositions of the present invention preferably are formulated as an aqueous solution for administration as an aerosol or nasal drops, or as a dry powder, e.g. for inhalation.

Compositions for administration as nasal drops may contain one or more excipients of the type usually included in such compositions, for example preservatives, viscosity adjusting agents, tonicity adjusting agents, buffering agents and the like.

The present invention also contemplates the provision of means for dispensing intranasal formulations of influenza antigen and mutant pertussis toxin. A dispensing device may, for example, take the form of an inhaler device or an aerosol delivery system, and may be arranged to dispense only a single dose, or a multiplicity of doses.

The vaccine will be administered to the patient in an amount effective to stimulate a protective immune response in the patient. For example, the vaccine may be administered to humans in one or more doses, each dose containing 1-250 microgrammes and more preferably 3-50 microgrammes of protein prepared from each virus strain. For example, where haemagglutinin and neuraminidase preparations are prepared from three virus strains, e.g. 2 × Influenza A and 1 × Influenza B, a total dose of viral protein administered could be in the range 9-150 microgrammes.

The invention will now be illustrated by way of example with reference to the specific embodiments described below and with reference to the accompanying drawings.

### BRIEF DESCRIPTION OF THE FIGURES

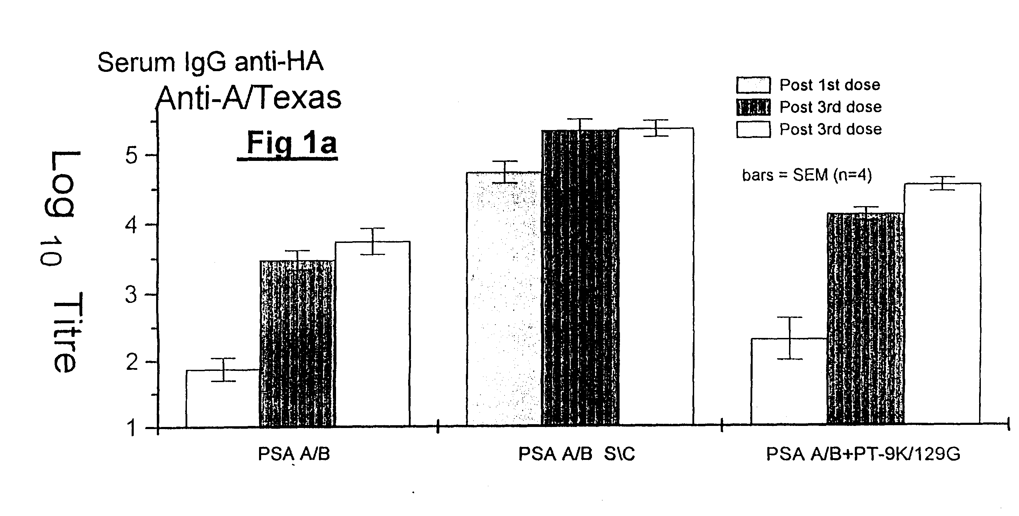

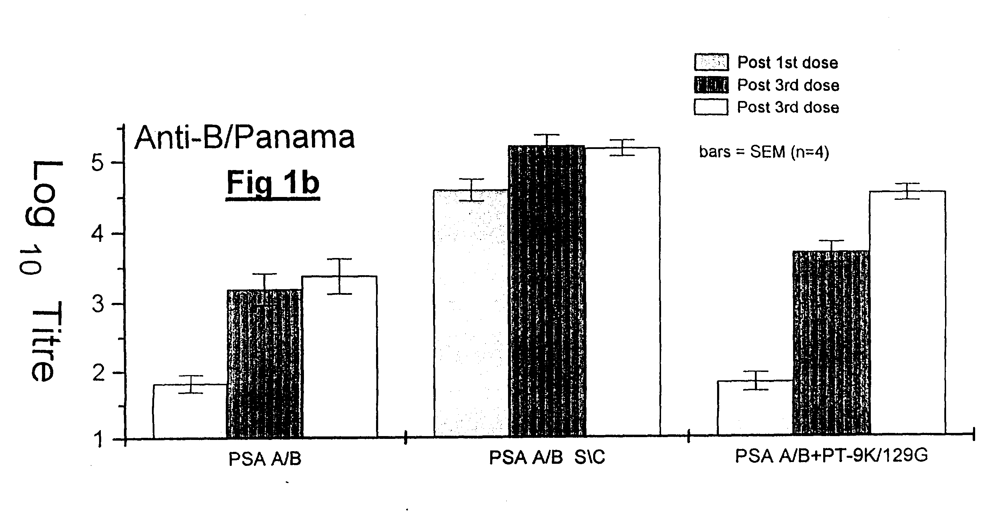

Figures 1a and 1b illustrate the serum IgG anti-haemagglutinin responses in mice immunised with purified surface antigen (PSA) derived from Influenza A Texas strain and PSA derived from Influenza B Panama strain, alone or in combination with the pertussis toxin mutant PT-9K/129G. Each bar represents the geometric mean titre of four mice. The error bars represent 1 standard error of the mean. The cut-off titre is 50 which is the lower limit of detection.

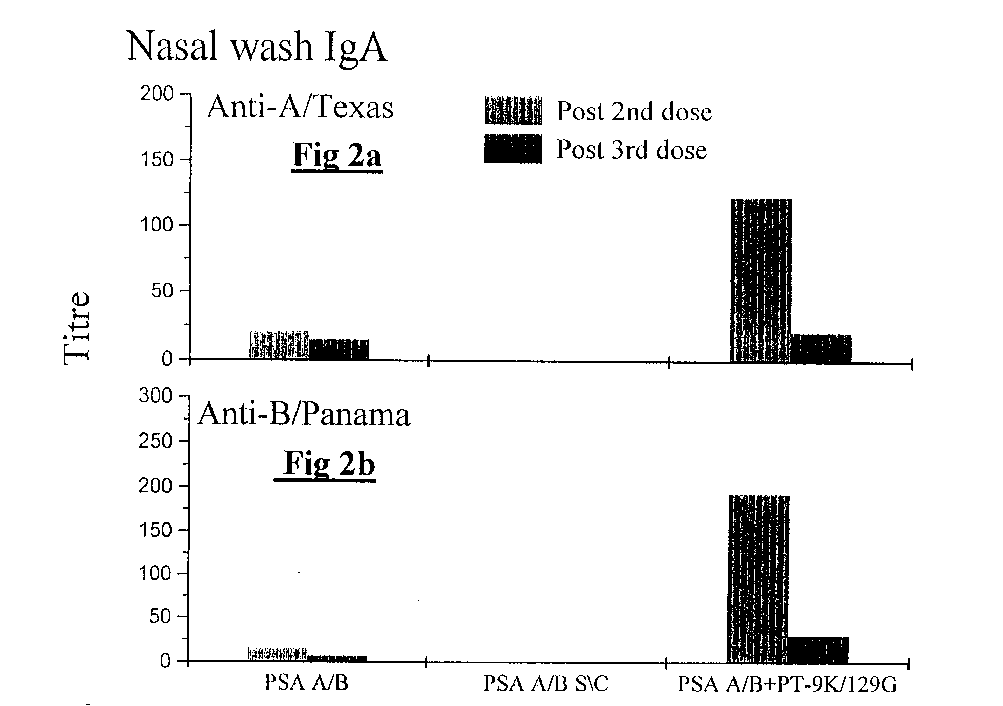

Figures 2a and 2b illustrate the nasal IgA anti-haemagglutinin responses in mice immunised with purified surface antigen (PSA) derived from the Influenza A Texas strain and the Influenza B Panama strain, alone or in combination with PT-9K/129G. As with Figure 1a and 1b, each bar represents the geometric mean titre of four mice, and the error bars represent 1 standard error of mean.

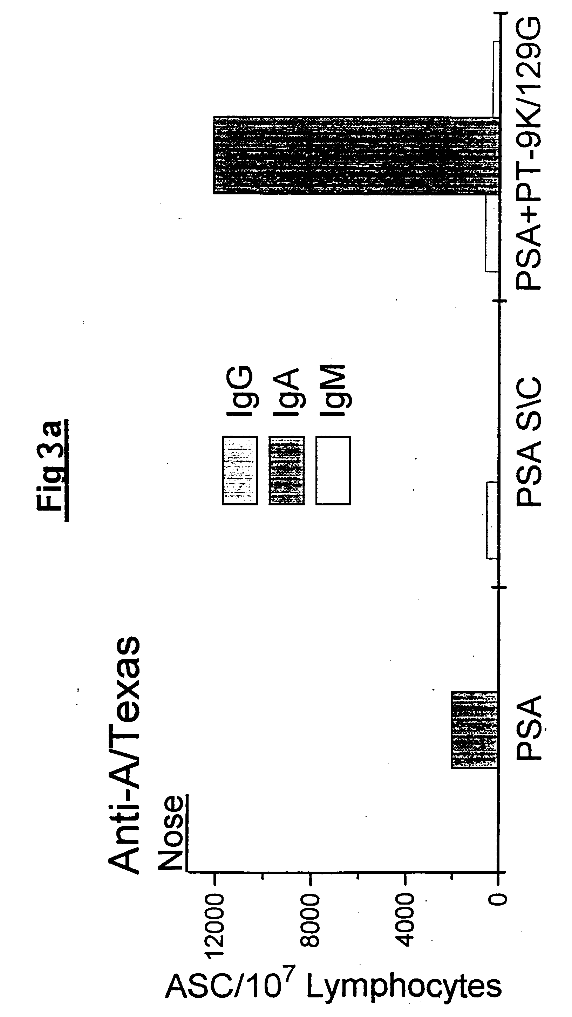

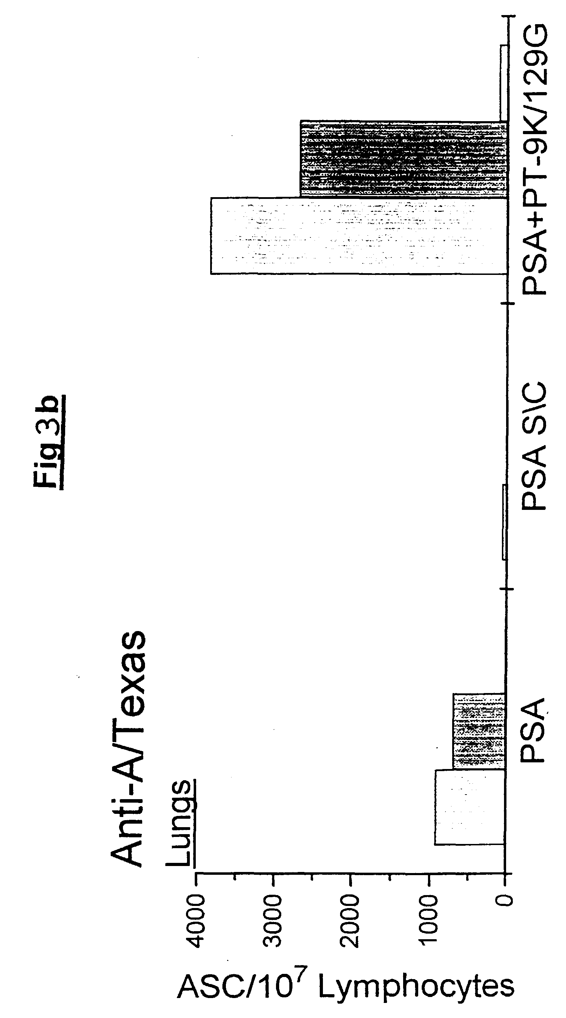

Figures 3a and 3b illustrate the determination, using the ELISPOT technique, of nasal and pulmonary anti-haemagglutinin (strain A Texas) secreting cells of mice immunised with purified surface antigen (PSA) derived from Influenza A Texas strain and the Influenza B Panama strain, alone or in combination with PT-9K/129G.

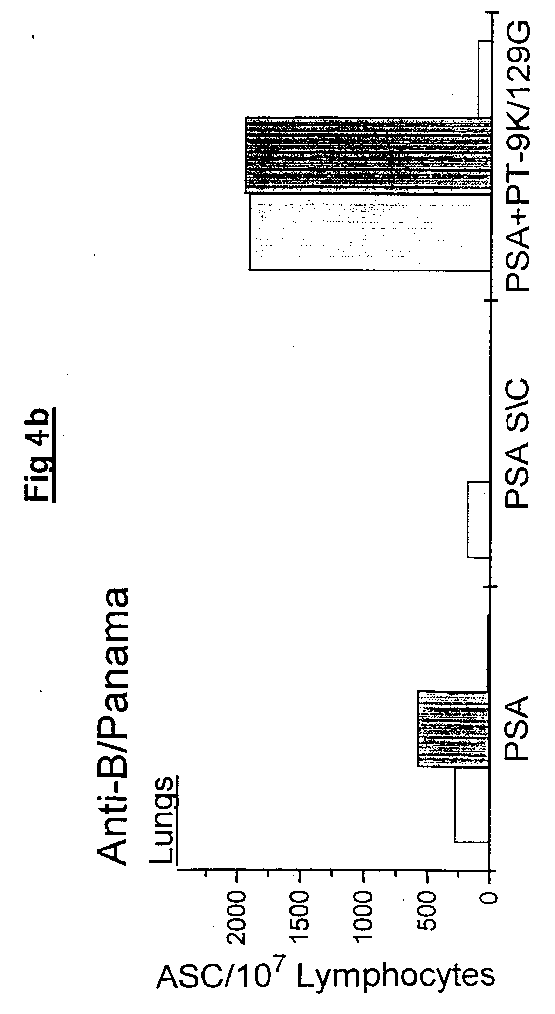

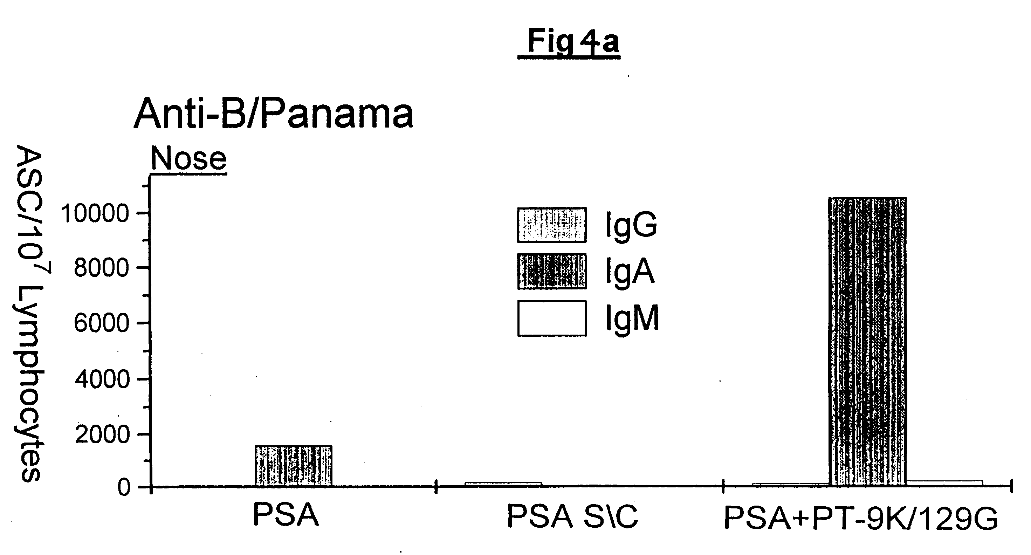

Figures 4a and 4b illustrate the determination, using the ELISPOT technique, of the nasal and pulmonary anti-haemagglutinin (strain B Panama) secreting cells of mice immunised with PSA derived from Influenza A Texas strain and the Influenza B Panama strain, alone or in combination with PT-9K/129G.

### EXAMPLE 1

Influenza purified surface antigen (PSA) containing both Influenza A Texas strain and Influenza B Panama strain protein, commercially available from Evans Medical Limited, Speke, Merseyside, United Kingdom, under the Trade Mark "Fluvirin", was made up in phosphate buffered saline along with B. pertussis toxin mutant PT-9K/129G, obtained from IRIS Biocine, of Siena, Italy (4.4mg/dose) to give a total influenza antigen protein concentration of approximately 1mg/ml and a pertussis toxin protein concentration of 0.25 mg/ml. The influenza PSA consists almost entirely of the spike protein haemagglutinin (HA), although it does contain some neuraminidase.

Control solutions containing the same concentrations of PSA but not PT-9K/129G, were also prepared. In addition, a composition comprising the same concentration of PSA adsorbed on to the known adjuvant alhydrogel (aluminium hydroxide) was prepared. The PSA was adsorbed on to the alhydrogel overnight at 4°C.

### Mice Immunisation Studies

The compositions prepared as described above were administered to groups of twelve adult (6-8 weeks) female BALB/c mice as follows:
- Group 1.: 20µl (10µl per nostril) PSA (A+B)/PT-9K/129G solution administered intranasally (total PSA dose = 20µg). PT-9K129G=5µg
- Group 2.: 20µl PSA administered intranasally (total PSA dose = 20µg) .
- Group 3.: 200µl PSA (A+B)/alhydrogel administered subcutaneously (total PSA dose = 20µg).

The immunisation procedure was carried out three times at approximately monthly intervals, as shown in the immunisation and sampling regime in Table 1.

### Immunisation and sampling regime

**TABLE 1**

| **Immunisation** | **Day** | **Sample** | **Day** |
|---|---|---|---|
| 1 | 1 | 1 | 21 |
| 2 | 28 | 2 | 42 |
| 3 | 61 | 3 | 77+79 |

At each sampling point four mice from each group were terminally bled by cardiac puncture, their heads were removed and their nasal passages lavaged with 1ml PBS + 1% bovine serum albumin.

### Antibody assays

In all assays whole influenza vaccine (WIV) strains A Texas and B Panama was used as antigen. Although WIV is only ∼50% HA the assays were thought to be measuring primarily anti-HA antibodies. This assumption was confirmed by substituting PSA (∼100% HA) for WIV and repeating some assays. The results were similar with either antigen. HA-specific serum IgG and nasal IgA antibodies were measured by Enzyme Linked Immunosorbant Assay (ELISA). After correcting for background, the individual optical density (OD) dilution curves were plotted and the titre values determined. The titre was determined as the dilution of serum that gave an OD reading of 0.2 or the dilution of nasal wash that gave an OD reading of O.1.

As well as taking nasal washes at the third sample lymphocytes were isolated from the mucous membranes of the nasal cavity and the lungs and the local immune response analysed by ELISPOT.

### Results

### 1. Serum anti-HA serum response

### Purified Surface Antigen (Figure 1 and Table 2 and 3)

As expected a good serum response was elicited by subcutaneous (S\C) immunisation with PSA + Alhydrogel. All the animals tested had seroconverted after the primary immunisation and the geometric mean titre (GMT) was good. The response increased after each boost in the case of Influenza A Texas and the GMT after the third dose was very high (∼230,000). In the case of Influenza B Panama, the GMT after the third dose had decreased slightly from the level obtained after the first dose but was nevertheless still high (ca 150,000). In contrast the serum response to PSA alone administered intranasally was poor: in the case of Influenza B Panama, only one of four mice had seroconverted after the first dose, and although all animals tested had seroconverted after the third dose the GMT was much lower than that of animals receiving one dose of PSA + Alhydrogel. PT-9K/129G (referred to in the table for convenience as "crm" enhanced the serum response of intranasally administered PSA; after the third vaccination the anti-A/Texas antibody response in mice that received PSA + PT-9K/129G was approximately six times that of mice receiving PSA alone I\N, and the anti-B/Panama response was approximately fifteen times that of mice receiving PSA alone I/N.

**TABLE 2**

| **Serum IgG anti-A/Texas response: effect of PT-9K/129G** | | | | | | |
|---|---|---|---|---|---|---|
| **Group** | **Post-Dose 1** | | **Post-Dose 2** | | **Post-Dose 3** | |
| | **Seroconversion** | **GMT**^{**b**} | **Seroconversion** | **GMT**^{**b**} | **Seroconversion** | **GMT**^{**b**} |
| PSA A/B I/N | 4/4^{a} | 75 | 4/4 | 2986 | 4/4 | 5518 |
| PSA A/B S/C | 4/4 | 53465 | 4/4 | 218716 | 4/4 | 232511 |
| PSA A/B crm | 3/4 | 203 | 4/4 | 13048 | 4/4 | 34526 |

| | | | | | | |
|---|---|---|---|---|---|---|
| ^{a} No. positive/No. tested | | | | | | |
| ^{b} Geometric Mean Titre | | | | | | |

**TABLE 3**

| **Serum IgG anti-B/Panama response: effect of PT-9K/129G** | | | | | | |
|---|---|---|---|---|---|---|
| Group | Post Dose 1 | | Post Dose 2 | | Post Dose 3 | |
| | Serocon. | GMT^{b} | Serocon. | GMT^{b} | Serocon. | GMT^{b} |
| PSA A/B I/N | 1/4 | 66 | 3/4 | 1,528 | 4/4 | 2,368 |
| PSA A/B S/C | 4/4 | 38,538 | 4/4 | 163,372 | 4/4 | 149,607 |
| PSA A/B +crm | 1/3 | 67 | 4/4 | 4,967 | 4/4 | 33,971 |

| | | | | | | |
|---|---|---|---|---|---|---|
| ^{a} No. positive/No. tested ^{b} Geometric Mean Titre | | | | | | |

### 2. Nasal wash IgA anti-HA response

### Purified Surface Antigen (Figure 2 and Table 4 and 5)

PSA + Alhydrogel given subcutaneously was very poor at inducing a nasal IgA response against either Influenza A Texas strain or Influenza B Panama strain which is consistent with our previous findings and those of others. PSA alone given intranasally was also a poor mucosal immunogen although it was better than subcutaneous immunisation in terms of the number of animals producing IgA. Adding PT-9K/129G significantly enhanced the IgA response to both the Influenza B Panama and Texas A strain. This response was boosted greatly in these mice by the second immunisation, and was also slightly boosted after the third immunisation. The final immunisation had little effect; in fact the mean specific IgA levels had decreased slightly.

**TABLE 4**

| **Nasal IgA anti-A/Texas response: effect of PT-9K/129G** | | | | |
|---|---|---|---|---|
| **Group** | **Post-Dose 2** | | **Post-Dose 3** | |
| | **IgA conversion** | **GMT**^{**b**} | **IgA conversion** | **GMT**^{**b**} |
| PSA A/B I/N | 4/4^{a} | 21 | 4/4 | 15 |
| PSA A/B S/C | 0/4 | <1 | 0/4 | 1 |
| PSA A/B +crm | 4/4 | 123 | 4/4 | 21 |

| | | | | |
|---|---|---|---|---|
| ^{a} No. positive\No. tested | | | | |
| ^{b} Geometric Mean Titre | | | | |

**TABLE 5**

| **Nasal IgA anti-B/Panama response: effect of PT-9K129G** | | | | |
|---|---|---|---|---|
| **Group** | **Post Dose 2 IgA** | | **Post Dose 3 IgA** | |
| | **Conver.** | **GMT** | **Convers.** | **GMT** |
| **PSA A/B I/N** | **4/4** | **16** | **4/4** | **7** |
| **PSA A/B S/C** | **0/4** | **1** | **0/4** | **1** |
| **PSA A/B +crm** | **4/4** | **195** | **4/4** | **31** |
| ^{a} No. positive\No. tested | | | | |
| ^{b} Geometric Mean Titre | | | | |

### Local anti-HA antibody secreting cell response (ASC) in nasal and pulmonary tissues

Lymphocytes were isolated from the nasal mucosa and lung parenchyma of groups of four mice from all groups at the third sampling point. Lymphocytes from individual mice were pooled and assayed for cells secreting IgA, IgG and IgM anti-flu antibodies using ELISPOT. The results are shown in Figures 3 and 4.

B cells secreting HA-specific antibodies were detectable in the nasal and lung tissue of all groups. There were far greater number of such cells in the PSA + PT-9K/129G groups for both Influenza A Texas and Influenza B Panama, and this is most apparent when the results are plotted on a linear scale (Figure 3 and 4). In all cases, except subcutaneously immunised mice, IgA antibody secreting cells (ASC) predominated in the nasal cavity whereas IgG predominated in the lungs.

### ELISPOT assay for specific antibody secreting cells (ASC) in murine lungs

Local antibody production in the murine lung was determined using the ELISPOT technique. Lymphocytes were isolated from murine lungs as follows: Lungs were washed briefly in PBS to remove traces of blood and were then finely chopped with a scalpel blade. 1ml of PBS containing 10mM MgCl₂, 0.5U/ml collagenase A (Boehringer Mannheim, Lewes, UK) and 0.25m/ml DNase 1 (Boehringer) was added for each pair of lungs and incubated at 37 degrees C with gentle agitation for 45 minutes. The mixture was then passed through a 40 gauge mesh. Lumps were pressed through the mesh with the plunger from a 5ml syringe. The cell suspension was placed in a centrifuge tube and allowed to stand for several minutes to allow large debris to settle. The supernatant was removed and the cells were pelleted and washed several times. Red cells and non-viable cells were removed by centrifugation on a Ficol-Isopaque gradient (LSM, Flow Laboratories Ltd, Herts, UK). After washing, cell viability was determined by Trypan Blue exclusion. Cells were finally suspended in RPM11640 complete medium (10% foetal calf serum, penicillin 100IU/ml, streptomycin 100ug/ml, L-glutamine 2mg: Flow).

The ELISPOT assay was performed as follows. Briefly, 24-well tissue culture plates (Costar) were coated overnight with the influenza antigen. After washing and blocking, 0.5ml volumes of dilutions of the lymphocyte suspension in complete RPM11640 were added to the wells and incubated at 37 degrees C/10% CO₂ for three hours. After washing, goat anti-mouse IgG, A or M (1/1000, Sigma) and rabbit anti-goat IgG-alkaline phosphatase (1/1000, Sigma) were added sequentially. Finally, substrate solution (0.5ul of 1mg/ml 5-bromo-4-chloro-3-indolyl phosphate (BCIP) in 2-amino-2-methyl-1,3-propanediol (AMP) buffer (Sigma) was added and plates were incubated until blue spots were visible under low power microscopy.

## Claims

1. A vaccine composition comprising an effective immunogenic amount of an influenza antigen and an effective adjuvant amount of a non-toxic mutant form of pertussis toxin, wherein both influenza A and influenza B antigens are present in the composition.

2. A vaccine composition according to claim 1 wherein the influenza antigen comprises haemagglutinin and neuraminidase antigens.

3. A vaccine composition according to claim 2 wherein the influenza antigen comprises purified haemagglutinin and neuraminidase antigens.

4. A vaccine composition according to claim 3 wherein the purified haemagglutinin and neuraminidase antigens are in the form of rosettes.

5. A vaccine composition according to claim 4 wherein the rosettes are particles with a radius in the range from 10 to 25 nanometres.

6. A vaccine composition according to claim 4 or claim 5 wherein the rosettes are substantially free of lipids.

7. A composition according to any one of the preceding Claims wherein the non-toxic mutant form of pertussis toxin is one in which the Glu 129 amino acid in the S₁ sub-unit has been substituted by another amino acid.

8. A composition according to claim 7 wherein the Glu 129 amino acid has been substituted by glycine.

9. A composition according to any one of the preceding Claims wherein the non-toxic mutant form of pertussis toxin is one in which the arginine 9 amino acid residue has been substituted by another amino acid.

10. A composition according to Claim 9 wherein the arginine 9 amino acid has been substituted by lysine.

11. A composition according to any one of the preceding claims which is adapted for mucosal administration.

12. A composition according to claim 11 which is adapted for intranasal administration.

13. A pharmaceutical product comprising a dispensing device adapted to deliver a composition intranasally, in combination with a vaccine composition as defined in any one of the preceding Claims.

14. A pharmaceutical product according to Claim 13 wherein the dispensing device is an aerosol delivery system.

15. The use of a non-toxic mutant form of pertussis toxin as defined in any one of the preceding claims for the manufacture of an adjuvant composition for stimulating or enhancing the mucosal immunogenicity of an influenza antigen co-administered therewith, wherein the composition contains both influenza A and influenza B antigens.

## Patentansprüche

1. Impfstoffzusammensetzung, die eine wirksame immunisierende Menge eines Influenza-Antigens und eine wirksame unterstützende Menge einer nicht toxischen Abart eines Pertussis-Toxins enthält, wobei sowohl Influenza A- als auch Influenza B-Antigene in der Zusammensetzung enthalten sind.

2. Impfstoffzusammensetzung nach Anspruch 1, wobei das Influenza-Antigen Hämagglutinin- und Neuraminidase-Antigene enthält.

3. Impfstoffzusammensetzung nach Anspruch 1, wobei das Influenza-Antigen gereinigte Hämagglutinin- und Neuraminidase-Antigene enthält.

4. Impfstoffzusammensetzung nach Anspruch 3, wobei die gereinigten Hämagglutinin- und Neuraminidase-Antigene Rosettenform aufweisen.

5. Impfstoffzusammensetzung nach Anspruch 4, wobei die Rosetten Partikel mit einem Radius zwischen 10 nm und 25 nm sind.

6. Impfstoffzusammensetzung nach Anspruch 4 oder 5, wobei die Rosetten im wesentlichen frei von Lipiden sind.

7. Impfstoffzusammensetzung nach einem der vorangehenden Ansprüche, wobei in der nicht toxischen Abart des Pertussis-Toxins die Glutaminsäure 129-Aminosäure in der S₁-Untereinheit durch eine andere Aminosäure ersetzt ist.

8. Impfstoffzusammensetzung nach Anspruch 7, wobei die Glutaminsäure 129-Aminosäure durch Glycin ersetzt ist.

9. Impfstoffzusammensetzung nach einem der vorangehenden Ansprüche, wobei in der nicht toxischen Abart des Pertussis-Toxins der Arginin 9-Aminosäurerest durch eine andere Aminosäure ersetzt ist.

10. Impfstoffzusammensetzung nach Anspruch 9, wobei der Arginin 9-Aminosäurerest durch Lysin ersetzt ist.

11. Impfstoffzusammensetzung nach einem der vorangehenden Ansprüche zur mukosalen Verabreichung.

12. Impfstoffzusammensetzung nach einem der vorangehenden Ansprüche zur intranasalen Verabreichung.

13. Pharmazeutisches Produkt mit einem Spender zur intranasalen Verabreichung einer Zusammensetzung in Verbindung mit einer Impfstoffzusammensetzung nach einem der vorangehenden Ansprüche.

14. Pharmazeutisches Produkt nach Anspruch 13 wobei der Spender ein Aerosolerzeuger ist.

15. Verwendung eines Pertussis-Toxins nach einem der vorangehenden Ansprüche zur Herstellung einer unterstützenden Zusammensetzung zum Stimulieren oder Erhöhen der mukosal immunisierenden Wirkung eines mitverabreichten Influenza-Antigens, wobei die Zusammensetzung sowohl Influenza A- als auch Influenza B-Antigene enthält.

## Revendications

1. Une composition de vaccin comprenant une quantité immunogène efficace d'un antigène de la grippe et une quantité adjuvante efficace d'une forme mutante non toxique de la toxine de la coqueluche, dans laquelle à la fois les antigènes A et les antigènes B de la grippe sont présents dans la composition .

2. Une composition de vaccin selon la revendication 1 dans laquelle l'antigène de la grippe comprend des antigènes d'hémagglutinine et de neuramidase.

3. Une composition de vaccin selon la revendication 2 dans laquelle l'antigène de la grippe comprend des antigènes d'hémagglutinine et de neuramidase purifiés.

4. Une composition de vaccin selon la revendication 3 dans laquelle les antigènes purifiés d'hémagglutinine et de neuramidase sont sous la forme de rosettes.

5. Une composition de vaccin selon la revendication 4 dans laquelle les rosettes sont des particules ayant un rayon compris dans l'intervalle de 10 à 25 nanomètres.

6. Une composition de vaccin selon la revendication 4 ou la revendication 5 dans laquelle les rosettes sont en grande partie exemptes de lipides.

7. Une composition selon l'une quelconque des revendications précédentes dans laquelle la forme mutante non toxique de la toxine de la coqueluche est une forme dans laquelle l'acide aminé Glu 129 dans la sous-unité S1 a été remplacé par un autre acide aminé.

8. Une composition selon la revendication 7, dans laquelle l'acide aminé Glu 129 a été remplacé par la glycine.

9. Une composition selon l'une quelconque des revendications précédentes dans laquelle la forme mutante non toxique de la toxine de la coqueluche est une forme dans laquelle le résidu acide aminé arginine 9 a été remplacé par un autre acide aminé.

10. Une composition selon la revendication 9, dans laquelle l'acide aminé arginine 9 a été remplacé par la lysine.

11. Une composition selon l'une quelconque des revendications précédentes qui est adaptée pour une administration par voie muqueuse.

12. Une composition selon la revendication 11 qui est adaptée pour une administration intranasale.

13. Un produit pharmaceutique comprenant un dispositif de distribution adapté pour l'administration d'une composition par voie intranasale, en association avec une composition de vaccin telle que définie dans l'une quelconque des revendications précédentes.

14. Un produit pharmaceutique selon la revendication 13 dans lequel le dispositif de distribution est un système de distribution par aérosol.

15. L'utilisation d'une forme mutante non toxique de la toxine de la coqueluche tel que défini dans l'une quelconque des revendications précédentes pour la fabrication d'une composition adjuvante pour la stimulation ou l'accroissement de l'immunogénicité par voie muqueuse d'un antigène de la grippe co-administré, dans laquelle la composition contient à la fois les antigènes A et les antigènes B de la grippe .
